(19) Europäisches Patentamt
European Patent Office
Office européen des brevets

(11) Veröffentlichungsnummer: **0 057 825**
**B1**

(12) # EUROPÄISCHE PATENTSCHRIFT

(45) Veröffentlichungstag der Patentschrift: **03.10.84**

(51) Int. Cl.³: **C 07 D 323/00**

(21) Anmeldenummer: **82100284.7**

(22) Anmeldetag: **16.01.82**

(54) **Verfahren zur Herstellung aliphatischer cyclischer Kohlensäureester.**

(30) Priorität: **30.01.81 DE 3103137**

(43) Veröffentlichungstag der Anmeldung: **18.08.82 Patentblatt 82/33**

(45) Bekanntmachung des Hinweises auf die Patenterteilung: **03.10.84 Patentblatt 84/40**

(84) Benannte Vertragsstaaten: **CH DE FR GB IT LI**

(56) Entgegenhaltungen:
**EP - A - 0 013 957**
**EP - A - 0 013 958**
**DE - B - 1 229 101**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Krimm, Heinrich, Dr., Heyenbaumstrasse 65, D-4150 Krefeld 1 (DE)**
Erfinder: **Buysch, Hans-Josef, Dr., Brandenburger Strasse 28, D-4150 Krefeld 1 (DE)**

EP 0 057 825 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung cyclischer Kohlensäureester der aliphatischen Reihe ausgehend von Harnstoff.

Bekannte Herstellungsverfahren für cyclische Kohlensäureester bestehen in der Umsetzung von Bischlorkohlensäureestern mit Dihydroxyverbindungen (s. Makromolekulare Chemie *57*, 1) und in der Umsetzung von Alkandiolen mit Dialkylcarbonaten (J.A.C.S. *55*, 5031). Bischlorkohlensäureester und Dialkylcarbonate werden bislang technisch nur unter Verwendung von Phosgen hergestellt.

Im Interesse einer umweltfreundlichen Technologie besteht deshalb die Aufgabe, ein neues, die Verwendung von Phosgen vermeidendes Herstellungsverfahren für cyclische Carbonate zu entwickeln.

Es wurde nun ein Verfahren zur Herstellung aliphatischer cyclischer Kohlensäureester gefunden, das dadurch gekennzeichnet ist, dass man Harnstoff oder Carbamidsäureester mit mindestens der äquivalenten Menge eines aliphatischen Alkohols der Formel (I)

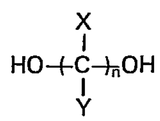

(I)

worin
X und Y sowohl innerhalb einer 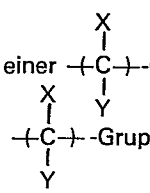 -Gruppe als auch bei verschiedenen -Gruppen gleich oder verschieden sein können und für Wasserstoff oder $C_1$-$C_4$-Alkyl stehen und
n für eine ganze Zahl von 3 bis 18 steht,
in Gegenwart von Thallium- oder Bleiverbindungen zunächst auf 140 bis 190°C, dann auf 200 bis 270°C erhitzt, bis sich kein Ammoniak mehr abspaltet und dann die aliphatischen cyclischen Kohlensäureester bei Drucken im Bereich 0,001 bis 50 mbar und Temperaturen im Bereich 150 bis 300°C aus dem Reaktionsgemisch entfernt.

Das erfindungsgemässe Verfahren kann letztlich ausgehend von Kohlendioxid, Ammoniak und einem aliphatischen Alkohol der Formel (I) durchgeführt werden, denn Harnstoff ist nach bekannten Verfahren aus Kohlendioxid und Ammoniak zugänglich [s. z.B. Ullmann, Encyclopädie der Technischen Chemie, 4. Auflage (1976), Band 12, S. 499 bis 502].

Anstelle von Harnstoff kann das erfindungsgemässe Verfahren auch ausgehend von Carbamidsäureestern durchgeführt werden, dem Umsetzungsprodukt von Harnstoff mit einem Alkohol.

Geht man von Harnstoff aus, so kann das erfindungsgemässe Verfahren, dargestellt mit Hexandiol-1,6 als Beispiel für einen aliphatischen Alkohol der Formel (I), durch folgende Gleichungen (1) bis (3) wiedergegeben werden:

$$2\ H_2N\text{-}CO\text{-}NH_2 \quad + \quad HO\text{-}(CH_2)_6OH \quad \rightarrow$$

$$H_2N\text{-}CO\text{-}O\text{-}(CH_2)_6O\text{-}CO\text{-}NH_2 \quad + \quad 2\ NH_3 \qquad (1)$$

$$H_2N\text{-}CO\text{-}O\text{-}(CH_2)_6O\text{-}CO\text{-}NH_2 + HO\text{-}(CH_2)_6OH \rightarrow$$

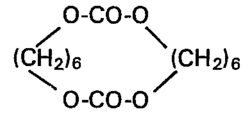

$+ \quad 2\ NH_3 \qquad (2)$

Für die Gesamtreaktion gilt dann:

$$2\ NH_2\text{-}CO\text{-}NH_2 \quad + \quad 2\ HO\text{-}(CH_2)_6OH \quad \rightarrow$$

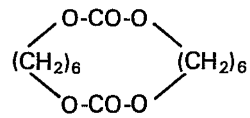

$+ \quad 4\ NH_3 \qquad (3)$

Danach werden gemäss Gleichung (1) aus Harnstoff zunächst Carbamidsäureester als Zwischenprodukte gebildet.

Dies ist eine seit langem bekannte Reaktion, die auch mit einwertigen Alkoholen möglich ist und dann zu Carbamidsäureestern einwertiger Alkohole führt, wie in Gleichung (4) erläutert:

$$H_2N\text{-}CO\text{-}NH_2 + HOR \rightarrow H_2N\text{-}CO\text{-}OR + NH_3 \qquad (4)$$

Als Katalysatoren für die Bildung von Carbamidsäureestern sind beispielsweise Verbindungen von Zink, Zinn, Blei und/oder Cobalt wie Zinkacetat, Zinntetrachlorid, Bleiacetat und Cobaltchlorid bekannt. Die Reaktionstemperaturen können beispielsweise 140 bis 190°C betragen.

Die erfindungsgemässe Umsetzung von Carbamidsäureestern zweiwertiger Alkohole bzw. der zweite Teil der erfindungsgemässen Umsetzung von Harnstoff mit Alkandiolen der Formel (I), bedarf höherer Temperaturen und längerer Reaktionszeiten.

Als aliphatische Alkohole der Formel (I) kommen vorzugsweise geradkettige aliphatische Alkandiole mit 3 bis 12 C-Atomen, beispielsweise Propandiol-1,3, Butandiol-1,4, Pentandiol-1,5, Hexandiol-1,6, Octandiol-1,8, Decandiol-1,10 und Dodecandiol-1,11, verzweigte aliphatische Diole mit 4 bis 12 C-Atomen, beispielsweise 2,2-Dimethyl-propandiol-1,3 (= Neopentylglykol), 2,2-Diethyl-propandiol-1,3, Trimethylpentandiol und Trimethylhexandiol-1,6, in Frage.

Die Mindestmenge einzusetzenden aliphatischen Alkohols der Formel (I) richtet sich danach, ob Harnstoff oder ein Carbamidsäureester in das erfindungsgemässe Verfahren eingesetzt wird. Im ersten Fall ist mindestens 1 Mol, im zweiten Fall mindestens ein halbes Mol des betreffenden Diols pro Carbamidsäureäquivalent einzusetzen. Wird also ein bifunktioneller Carbamidsäureester gemäss Gleichung (1) eingesetzt, ist mindestens 1 Mol Diol erforderlich. Wird ein monofunktioneller Carbamidsäureester eingesetzt [s. Gleichung (4)], ist mindestens ein halbes Mol Diol erforderlich.

Für das erfindungsgemässe Verfahren geeignete Carbamidsäureester sind Biscarbamidsäureester, die sich von den zweiwertigen Diolen der Formel (I) ableiten und Carbamidsäureester, die sich von einwertigen primären aliphatischen Alkoholen mit beispielsweise 4 bis 12 C-Atomen ableiten. Beispiele für

solche einwertigen primären aliphatischen Alkohole sind Hexanol, Octanol und 2-Ethylhexanol.

Es ist vorteilhaft, beispielsweise im Hinblick auf einen möglichst quantitativen Umsatz des Harnstoffs bzw. Carbamidsäureesters und auf einen schnellen Reaktionsablauf, einen Überschuss an Alkoholäquivalenten anzuwenden, beispielsweise doppelt bis viermal soviel Alkoholäquivalente als die oben angegebenen Mindestmengen und/oder zusätzlich einen einwertigen primären aliphatischen Alkohol mit 4 bis 12 C-Atomen zuzufügen, beispielsweise 1 Mol eines solchen Alkohols pro Mol Harnstoff bzw. Carbamidsäureester, wobei als Beispiele für solche einwertigen primären aliphatischen Alkohole, Hexanol, Octanol und 2-Ethylhexanol genannt seien.

Die erfindungsgemässe Umsetzung erfolgt in Gegenwart von Verbindungen des Thalliums oder Bleis als Umesterungskatalysatoren. Vorzugsweise wird als Umesterungskatalysator Thalliumhydroxid, -oxid, -methylat, -acetat, -naphthenat, -carbonat oder Blei-(II)-oxid, -acetat oder -naphthenat eingesetzt. Die Umesterungskatalysatoren können beispielsweise in einer Menge von 0,001 bis 1 Gew.-%, vorzugsweise von 0,01 bis 0,1 Gew.-%, jeweils bezogen auf das Reaktionsgemisch, eingesetzt werden.

Die Reaktionstemperatur beträgt beim erfindungsgemässen Verfahren zunächst 140 bis 190°C, anschliessend 200 bis 270°C, vorzugsweise 220 bis 260°C. Dabei entwickelt sich Ammoniak. Das Erhitzen auf diese Temperaturen wird so lange durchgeführt, bis sich kein Ammoniak mehr abspaltet, was beispielsweise 10 bis 20 Stunden dauern kann. Wenn ausser Ammoniak noch weitere bei diesen Temperaturen flüchtige Verbindungen im Reaktionsgemisch enthalten sind, beispielsweise niedrig siedende Alkohole, so wird zweckmässigerweise dem Reaktionsgefäss ein Kühler oder eine Kolonne aufgesetzt, um die gewünschte Reaktionstemperatur aufrechterhalten zu können. Der sich abspaltende Ammoniak kann in einer Kühlfalle oder in einer wässrigen Säurelösung aufgefangen werden.

Nachdem die Abspaltung von Ammoniak unter den vorstehenden Bedingungen beendet ist, werden zweckmässigerweise niedriger als die sich bildenden aliphatischen cyclischen Kohlensäureester siedende Bestandteile des Reaktionsgemisches durch Destillation bei vermindertem Druck abgetrennt, sofern solche Bestandteile vorhanden sind. Derartige Bestandteile können beispielsweise im Überschuss eingesetzte Alkandiole der Formel (I) oder eingesetzte einwertige, primäre, aliphatische Alkohole sein. Es ist möglich, dass sich bei dieser Abtrennung noch Restmengen von Ammoniak abspalten.

Die erfindungsgemäss zugänglichen aliphatischen cyclischen Kohlensäureester werden dann erhalten, indem man das verbleibende Reaktionsgemisch auf Temperaturen im Bereich 150 bis 300°C, vorzugsweise 210 bis 260°C erhitzt und dann bei Drucken im Bereich von 0,001 bis 50 mbar, vorzugsweise von 0,01 bis 1 mbar, aus dem Reaktionsgemisch entfernt. Diese Entfernung kann durch Destillation oder Sublimation erfolgen. Vorzugsweise stellt man innerhalb der angegebenen Druck- und Temperaturgrenzen solche Bedingungen ein, dass die Entfernung durch Destillation erfolgt.

Die so erhaltenen cyclischen Kohlensäureester fallen als definierte, vielfach kristallisierende Verbindungen an und können, falls dies gewünscht wird, noch gereinigt werden. Diese Reinigung kann beispielsweise durch Umkristallisation aus einem geeigneten Lösungsmittel, beispielsweise Essigsäureethylester, Toluol, Xylol und Ligroin, oder durch nochmalige Destillation erfolgen.

Die so erhaltenen aliphatischen cyclischen Kohlensäureester enthalten im allgemeinen 1 oder 2 Kohlensäureestergruppen und können der Formel (II)

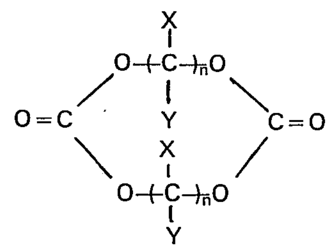

(II)

und/oder Formel (III)

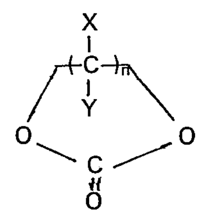

(III)

entsprechen, worin

X, Y und n die bei Formel (I) angegebene Bedeutung haben.

Überwiegend dimere Carbonate der Formel (II) werden erhalten, wenn die funktionellen Gruppen der Alkandiole der Formel (I) durch eine gerade Kette von 4 bis 8 C-Atomen verknüpft sind. Überwiegend monomere Carbonate der Formel (III) werden erhalten, wenn die funktionellen Gruppen der Alkandiole der Formel (I) durch eine gerade Kette von 3 oder von mehr als 11 C-Atomen verknüpft sind.

Die erfindungsgemäss zugänglichen aliphatischen cyclischen Kohlensäureester können in bekannter Weise verwendet werden, beispielsweise zur Herstellung von hoch-molekularen Polycarbonaten ohne Endgruppen [s. z.B. J.A.C.S. *55*, 5031 bis 5039 (1933)]. Diese können als Weichmacher, Lacke und Kunststoffe verwendet werden.

*Beispiele*

*Beispiel 1*

104 g (1 Mol) Neopentylglykol, 60 g (1 Mol) Harnstoff, 130 g (1 Mol) Octanol und 0,05 g Thalliumcarbonat wurden in einer Stickstoffatmosphäre an einer 40-cm-Füllkörperkolonne unter Rühren auf 160°C erhitzt, während Ammoniak abgespalten und in 2n-Salzsäure aufgefangen wurde. Sowie die Ammoniakentwicklung nachliess, wurde die Innentemperatur erhöht. Nach 17 Stunden lag sie bei 240°C und 1,9 Mol Ammoniak hatten sich entwickelt. Nach Entfernung der Kolonne wurden bei 104 bis 150°C und

19 mbar 90 g Vorlauf abdestilliert. Unter dem gleichen Druck ging bei 158 bis 168°C Neopentylglykolcarbonat über. Es wurden 103 g Kristalle erhalten. Der Rest des Neopentylglykolcarbonats lag als Polymeres vor, das bei Temperaturen bis zu 250°C/19 mbar depolymerisiert wurde. Es wurden weitere 30 g überwiegend kristallines Produkt erhalten. Durch Umkristallisation aus Xylol wurden insgesamt 106 g Neopentylglykolcarbonat vom Schmelzpunkt 109 bis 111°C erhalten. Die Ausbeute betrug 82% der Theorie. Aus der Mutterlauge wurden durch Destillation 19 g Dioctylcarbonat vom Schmelzpunkt 200 bis 202°C bei 16 mbar abgetrennt.

*Beispiel 2*

30 g des Ringcarbonats aus Neopentylglykol, das entsprechend Beispiel 1 erhalten wurde, wurde nach Zugabe von 30 mg Lithiumbenzoat unter Rühren auf 140°C erhitzt. Nach 10 Minuten wurde die Schmelze so steif, dass der Rührer nicht mehr arbeiten konnte. Die Temperatur erreichte 158°C. Nach Abkühlen auf Raumtemperatur wurde ein opakes, zähes Polymerisat erhalten. Aus den Lösungen in Methylenchlorid liess sich ein transparenter Film von grosser Reissfestigkeit giessen.

## Patentansprüche

1. Verfahren zur Herstellung aliphatischer cyclischer Kohlensäureester, dadurch gekennzeichnet, dass man Harnstoff oder Carbamidsäureester mit mindestens der äquivalenten Menge eines aliphatischen Alkohols der Formel

$$HO-\left(\overset{\displaystyle X}{\underset{\displaystyle Y}{C}}\right)_n-OH$$

worin

X und Y sowohl innerhalb einer $-\left(\overset{X}{\underset{Y}{C}}\right)-$-Gruppe als auch bei verschiedenen $-\left(\overset{X}{\underset{Y}{C}}\right)-$-Gruppen gleich oder verschieden sein können und für Wasserstoff oder $C_1$-$C_4$-Alkyl stehen und

n für eine ganze Zahl von 3 bis 18 steht,

in Gegenwart von Thallium- oder Bleiverbindungen zunächst auf 140 bis 190°C, dann auf 200 bis 270°C erhitzt, bis sich kein Ammoniak mehr abspaltet, und dann die aliphatischen cyclischen Kohlensäureester bei Drucken im Bereich 0,001 bis 50 mbar und Temperaturen im Bereich 150 bis 300°C aus dem Reaktionsgemisch entfernt.

2. Verfahren nach Anspruch 1, dadurch gekennzeichnet, dass man Harnstoff einsetzt.

3. Verfahren nach Anspruch 1, dadurch gekennzeichnet, dass man Harnstoff, einen aliphatischen Alkohol der Formel

$$HO-\left(\overset{\displaystyle X}{\underset{\displaystyle Y}{C}}\right)_n-OH$$

worin

X und Y sowohl innerhalb einer $-\left(\overset{X}{\underset{Y}{C}}\right)-$-Gruppe als auch bei verschiedenen $-\left(\overset{X}{\underset{Y}{C}}\right)-$-Gruppen gleich oder verschieden sein können und für Wasserstoff oder $C_1$-$C_4$-Alkyl stehen und

n für eine ganze Zahl von 3 bis 18 steht,

und einen einwertigen, primären, aliphatischen Alkohol mit 4 bis 12 C-Atomen einsetzt.

4. Verfahren nach Anspruch 1, dadurch gekennzeichnet, dass man als Carbamidsäureester Umsetzungsprodukte von Harnstoff mit einem Alkohol einsetzt.

5. Verfahren nach Ansprüchen 1 bis 4, dadurch gekennzeichnet, dass man als aliphatischen Alkohol der angegebenen Formel ein geradkettiges aliphatisches Alkandiol mit 3 bis 12 C-Atomen oder ein verzweigtes aliphatisches Diol mit 4 bis 12 C-Atomen einsetzt.

## Claims

1. Process for the preparation of aliphatic cyclic carbonates, characterised in that urea or carbamates are heated with at least an equivalent amount of an aliphatic alcohol of the formula

$$HO-\left(\overset{\displaystyle X}{\underset{\displaystyle Y}{C}}\right)_n-OH$$

wherein

X and Y can be identical or different not only within one $-\left(\overset{X}{\underset{Y}{C}}\right)-$ group but also for different $-\left(\overset{X}{\underset{Y}{C}}\right)-$ groups and represent hydrogen or $C_1$-$C_4$-alkyl and

n represents an integer from 3 to 18,

in the presence of thallium or lead compounds, first at 140 to 190°C, and then at 200 to 270°C, until no more ammonia is split off and then the aliphatic cyclic carbonates are removed from the reaction mixture under pressures in the range of 0.001 to 50 mbar and at temperatures in the range of 150 to 300°C.

2. Process according to Claim 1, characterised in that urea is used.

3. Process according to Claim 1, characterised in that urea, an aliphatic alcohol of the formula

$$HO-\left(\overset{\displaystyle X}{\underset{\displaystyle Y}{C}}\right)_n-OH$$

wherein

X and Y can be identical or different not only within one $-\left(\overset{X}{\underset{Y}{C}}\right)-$ group but also for different $-\left(\overset{X}{\underset{Y}{C}}\right)-$

groups and represent hydrogen or $C_1$-$C_4$-alkyl and n represents an integer from 3 to 18,
and a monohydric, primary aliphatic alcohol with 4 to 12 C atoms are used.

4. Process according to Claim 1, characterised in that products from the reaction of urea with an alcohol are used.

5. Process according to Claims 1 to 4, characterised in that a straight-chain aliphatic alkanediol with 3 to 12 C atoms or a branched aliphatic diol with 4 to 12 C atoms is used as the aliphatic alcohol of the formula indicated.

## Revendications

1. Procédé de préparation d'esters aliphatiques cycliques de l'acide carbonique, caractérisé en ce que l'on chauffe l'urée ou un ester carbamique avec au moins la quantité équivalente d'un alcool aliphatique de formule:

$$HO-\left(\underset{Y}{\overset{X}{C}}\right)_{n}-OH$$

dans laquelle
X et Y, aussi bien à l'intérieur d'un groupe $-\left(\underset{Y}{\overset{X}{C}}\right)-$ que dans le cas de plusieurs groupes $-\left(\underset{Y}{\overset{X}{C}}\right)-$ peuvent avoir des significations identiques ou différentes, et représentent l'hydrogène ou des groupes alkyle en $C_1$-$C_4$, et
n est un nombre entier de 3 à 18,
en présence de composés de thallium ou du plomb, d'abord à des températures de 140 à 190°C puis à des températures de 200 à 270°C, jusqu'à ce qu'il ne se sépare plus d'ammoniac, après quoi on élimine des esters aliphatiques cycliques de l'acide carbonique du mélange de réaction à des pressions dans l'intervalle de 0,001 à 50 mbar et des températures dans l'intervalle de 150 à 300°C.

2. Procédé selon la revendication 1, caractérisé en ce que l'on utilise l'urée.

3. Procédé selon la revendicatin 1, caractérisé en ce que l'on utilise l'urée, un alcool aliphatique de formule

$$HO-\left(\underset{Y}{\overset{X}{C}}\right)_{n}-OH$$

dans laquelle
X et Y, aussi bien à l'intérieur d'un groupe $-\left(\underset{Y}{\overset{X}{C}}\right)-$ que dans le cas de plusieurs groupes $-\left(\underset{Y}{\overset{X}{C}}\right)-$ peuvent avoir des significations identiques ou différentes et représentent l'hydrogène ou des groupes alkyle en $C_1$-$C_4$, et
n est un nombre entier de 3 à 18,
et un alcool aliphatique primaire monovalent en $C_4$-$C_{12}$.

4. Procédé selon la revendication 1, caractérisé en ce que l'on utilise en tant qu'esters carbamiques, des produits de réaction de l'urée et d'un alcool.

5. Procédé selon les revendications 1 à 4, caractérisé en ce que l'on utilise en tant qu'alcool aliphatique de formule ci-dessus, un alcane-diol aliphatique à chaîne droite en $C_3$-$C_{12}$ ou un diol aliphatique ramifié en $C_4$-$C_{12}$.